(19) Europäisches Patentamt
European Patent Office
Office européen des brevets

(11) **EP 1 638 616 B1**

(12) **EUROPEAN PATENT SPECIFICATION**

(45) Date of publication and mention
of the grant of the patent:
**10.03.2010 Bulletin 2010/10**

(51) Int Cl.:
***A61L 2/14*** *(2006.01)*

(21) Application number: **04763305.2**

(86) International application number:
**PCT/EP2004/007995**

(22) Date of filing: **30.06.2004**

(87) International publication number:
**WO 2005/002630 (13.01.2005 Gazette 2005/02)**

(54) **METHOD FOR DECONTAMINATION USING ATOMIC NITROGEN**

VERFAHREN ZUR DEKONTAMINATION MIT ATOMAREM STICKSTOFF

PROCEDE DE DECONTAMINATION UTILISANT DE L'AZOTE ATOMIQUE

(84) Designated Contracting States:
**AT BE BG CH CY CZ DE DK EE ES FI FR GB GR HU IE IT LI LU MC NL PL PT RO SE SI SK TR**

(30) Priority: **30.06.2003 US 610158**

(43) Date of publication of application:
**29.03.2006 Bulletin 2006/13**

(73) Proprietors:
• **CENTRE NATIONAL DE LA RECHERCHE SCIENTIFIQUE (CNRS)**
**75794 Paris Cedex 16 (FR)**
• **Université Paris-Sud 11**
**91405 Orsay (FR)**

(72) Inventors:
• **GANCIU PETCU, Mihai**
**R-76451 Bucarest (RO)**
• **POINTU, Anne-Marie**
**F-75016 Paris (FR)**
• **LEGENDRE, Bernard**
**F-92290 Chatenay-Malabry (FR)**
• **ORPHAL, Johannes**
**F-91300 Massy (FR)**

• **VERVLOET, Michel**
**F-91440 Bures-sur-Yvette (FR)**
• **TOUZEAU, Michel**
**F-91400 Orsay (FR)**
• **YACOUBI, Najet**
**F-94600 Choisy-le-Roi (FR)**

(74) Representative: **Texier, Christian et al**
**Cabinet Régimbeau**
**20, rue de Chazelles**
**75847 Paris Cedex 17 (FR)**

(56) References cited:
**WO-A1-00/54819      WO-A1-02/070025**
**US-A- 3 383 163      US-A- 6 030 506**

• **PLASMA PHYSICS REPORTS, [Online] vol. 26, no. 9, 2000, pages 792-800, XP002304874 Retrieved from the Internet: URL:http://www.kar.net/~plasma/results/pub 04.pdf> [retrieved on 2004-11-10]**

Remarks:
The file contains technical information submitted after the application was filed and not included in this specification

EP 1 638 616 B1

## Description

<u>FIELD OF THE INVENTION</u>

The present invention is related to a method for decontamination.

<u>BACKGROUND OF THE INVENTION</u>

Decontamination is performed in a wide range of ways, depending for example of the nature of the object to be decontaminated. In hospitals, for instance, the validated method for sterilization uses sealed autoclaves, at temperatures over 373K. Nevertheless, these temperatures can be harmful to those non-metallic materials which make up at least part of the objects to be decontaminated.

In another proposed decontamination / sterilization method, disclosed in FR-A-2 790 962, an active species having sporicidal effect is obtained from a mixture of $H_2O$, $N_2$ and $O_2$ and is transported in a region to be decontaminated. In this method, nevertheless, the use of water vapour in the decontaminating gas can cause the production of acid, which can be harmful to the materials to be decontaminated.

In the proceedings of club PISE "Stérilisation d'instruments médicaux par plasmas froids" ("Sterilization of medical apparatus by cold plasmas"), published October 19th, 2001, it is described, in "Post décharge en écoulement dans des tubes à la pression atmosphérique" ("Post-discharge in tubes at atmospheric pressure") an experiment wherein a discharge is created in a gas containing $N_2$ and $O_2$ and the afterglowing activated gas is propagated in a tube and exhibits UV fluorescence in an optical region that might be useful for decontamination. Nevertheless, the active species in this experiment is unknown and, would decontamination be provided by such a method, it would still be necessary to assess its potential effect on the materials of the decontamination area.

The article "Sterilization of Medical Products in Low-Pressure Glow Discharges" published in Plasma Physics Reports, 2000, relates to a decontamination method for decontamination of object containing spores of *Bacillus subtilis*. The method uses plasma composition containing molecule nitrogen or oxygen.

US Patent 3,383,163 concerns a sterilization treatment method for objects that might contain microorganisms such as bacteria and the like. The method comprises exposing the object to a pulsed plasma containing, for example, nitrogen.

US Patent 6,030,506 relates to a method and apparatus for performing large scale chemistry for bleaching, enhancing chemical reactions and pollution removal. The method uses plasma containing activated species which can be activated oxygen or nitrogen.

WO 00/54 819 relates to a method for sterilisation by plasma of an object in a treating chamber using a vector gas to propagate the generated plasma.

WO 02/070 025 relates to a method for plasma stérilisation at room temperature in the presence of moisture of a longitudinal object using a vector gas.

The paper STERILIZATION OF MEDICAL PRODUCTS IN LOW-PRESSURE GLOW DISCHARGES published in the PLASMA PHYSICS REPORTS by the AMERICAN INSTITUTE OF PHYSICS, NEW YORK, NY, US discloses a sterilisation method comprising the plasma-activation of pure nitrogen.

<u>OBJECTS AND SUMMARY OF THE INVENTION</u>

It is therefore an object of the present invention to provide an efficient decontamination method which could be harmless to the materials to be decontaminated.

To this end, the present invention provides a method for decontamination of a contaminated region containing at least biological species to be destroyed, according to here-enclosed claim 1.

Such biological species include for example cells, spores, bacteria, and others.

Nitrogen being dry, non-corrosive and not contributing to production of acid, it is therefore benign to most materials.

In various embodiments of the invention, one may use one and/or other of the of features of appended dependant claims.

<u>BRIEF DESCRIPTION OF THE DRAWINGS</u>

Other features of the invention will become apparent with the following detailed description of several embodiments thereof, illustrated by the accompanying drawing, in which:

- Fig. 1 is a schematic view of a decontamination apparatus according to a 1st embodiment of the invention,
- Fig. 2 is a schematic view of a decontamination apparatus according to a second embodiment of the invention,
- Fig. 3a is a schematic view of an atomic nitrogen generator according to the invention,
- Fig. 3b is a diagrammatic view of voltage pulses used in the generator, according to the invention, and
- Fig. 3c is a schematic view of an atomic nitrogen generator according to the invention.

On the various figures, similar or identical features are designated by the same numeral references.

<u>MORE DETAILED DESCRIPTION</u>

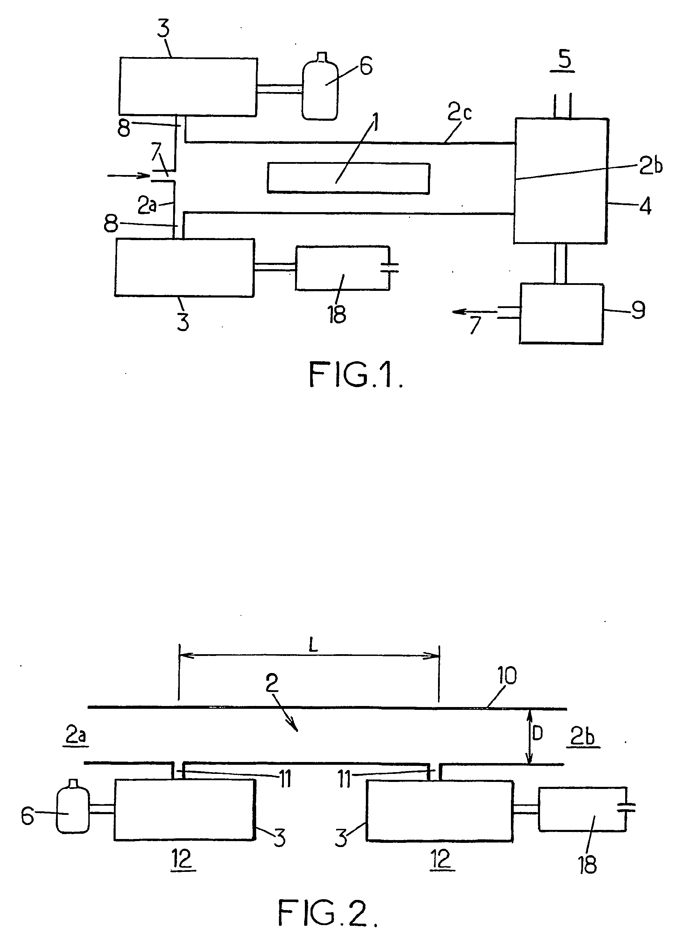

Fig. 1 represents a first application of the inventive method to decontamination of contaminated samples. Such samples can for example be medical apparatus too expensive to be discarded after a single utilisation, e.g. implants, endoscopes, catheters and similar small dimensions apparatus. The contaminated sample is not necessarily of medical type, and could for example be material used in food industry, papers, archaeological materials, or other suitable materials. Some particularly well-suited samples are those samples with intricate surface designs for which state of the art techniques do not prove efficient, like for example syringes.

By decontamination is understood here a destruction of biological species, such as cells, spores, bacteria, virus, micro-organisms, prions, fungus or others, most often deposited on the surface of the contaminated samples. Such decontamination can for example be a step in a sterilization process, or even a sterilization process on its own. Indeed, the imperative sporicidal effect for a sterilization process of achieving reduction of the concentration of bacteria by 12 decades can be achieved by the inventive method.

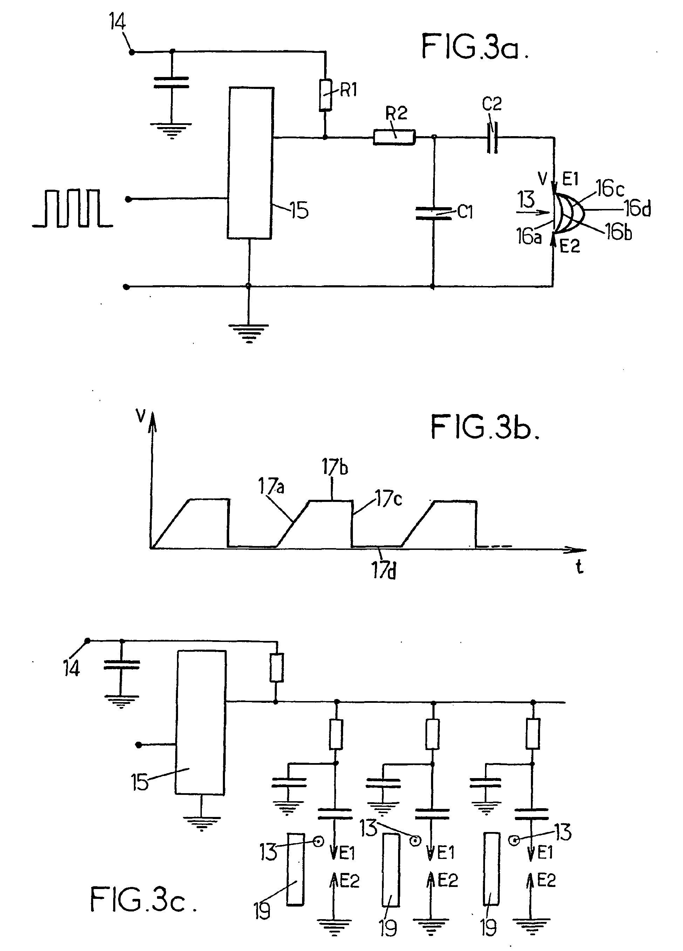

The contaminated sample 1 is placed in an adapted decontamination enclosure, for example cylindrical or hexahedral and of large enough dimensions to hold sample 1. Such a decontamination enclosure could for example consist of a one meter long and 20 cm wide cylindrical wall 2c, these dimensions being purely illustrative, ending on each side by respective first and second ends 2a, 2b. The internal wall of the decontamination enclosure is made of any suitable material such as glass, plastics, ceramics, metals or others. On first end 2a, the decontamination enclosure is connected to at least one atomic nitrogen generator 3 via an entrance duct 8. Such atomic nitrogen generator can for example be of the kind illustrated hereinafter with respect to Fig. 3, or any other suitable generator. On second end 2b, hereinafter referred to as exhaust end, the decontamination enclosure can for example be open to the ambient air 5, optionally through a filter 4 adapted to filter out the eventual products of the decontamination. The inside of said decontamination enclosure defines a contaminated space, with a high concentration of biological species to be destroyed.

Each generator can be connected to a source of gas 6 that emits a flow of gas containing gaseous molecular nitrogen towards the generator. The source gas can for example flow at around 500 $cm^3$/s. In addition to $N_2$, the source gas could for example contain other gas which have no decontamination effect but could be useful in other stages of an associated method.

$N_2$ of purity over 99% can be used in the scope of the present invention. Such $N_2$ is for example provided by a nitrogen jar. A molecular nitrogen generator 18 can also be used to generate a source gas with a controlled volume amount of $N_2$ for example over 99.99%. Such molecular nitrogen generators 18, for example Pressure Swing Adsorption nitrogen generators such as DYF (5-23) commercialized by Rich Air Separation Co. Ltd can be used to separate nitrogen from other gases contained in the air, using two beds of Carbon Molecular Sieve (CMS).

$N_2$ purity requirements can be decreased at lower operative temperatures, which might nevertheless require other operative settings, so that a trade-off appears the best solution depending on the intended application. Operative temperature can also be raised up to around 400K, if permitted by the decontaminated sample, so that decontamination properties of heat are added to the inventive method.

The source gas flows into the generator(s) 3 in which the gas is dissociated to provide nitrogen atoms such as $N(^4S)$, $N(^2D)$ and $N(^2P)$. Dissociation can for example occur under ambient conditions of temperature and pressure. If the contaminated sample 1 is made of thermo-sensible material, or for any other appropriate reason, the temperature of the gas in the generator can be kept under 373K at close-to-atmospheric pressure. The pressure of the vector gas can be adapted depending of the operative needs, in the range 6,6661-533,288 kPa (50-4000 Torr) for example.

As a result of the dissociation process in the generator 3, the flow of gas entering the contaminated region contains atomic nitrogen, for example in concentrations around $10^{14}$ $cm^{-3}$ to $10^{15}$ $cm^{-3}$. This concentration decreases with the distance z from the generator. At atmospheric pressure, vector gas temperature of 300K and purity of $N_2$ over 99.99%, the concentration [N] of atomic nitrogen as a function of z could be roughly described by following equation:

$$1/[N] = 1/[N_0] + k \cdot z,$$

where z is in centimetres, $[N_0]$ the concentration of atomic nitrogen close to the generator, and k is a constant depending on the flow of gas. Typical k values could be found between $10^{-n}$ and $10^{-15}$ $cm^2$. Thus, high atomic nitrogen concentration are maintained in the whole contaminated region.

The first end 2a of the decontamination enclosure optionally contains an intake duct 7 for a vector gas, which could for example flow at around 20 m/s into the decontamination enclosure, in a way that the vector gas intake duct 7 is close enough to the entrance ducts 8 through which decontamination gas enters contaminated region, in order for the vector gas to carry efficiently the decontamination gas throughout the contaminated region. Flow speed might range approximately between 1 m/s, which makes it possible for the active species to reach intricate parts of the contaminated sample 1, and 100 m/s, which makes it possible to keep high decontamination efficiency even far away from the intake.

Vector gas can for example be pure $N_2$, optionally the same gas as provided by the gas sources 6, or air filtered in intake duct 7 to contain no more than 0.1% $O_2$ in volume.

In addition, such gas containing $O_2$ can also be introduced into decontamination enclosure in the vicinity of contaminated sample 1. Thus, one can obtain in the decontamination enclosure species by reaction of atomic nitrogen and oxygen, such as $N(^4S)$, $N(^2D)$, $N(^2P)$, $N_2$ (B), $N_2$ (A), $O)(^3P)$, $O(^1D)$, $O(^1S)$, $O_2(^1\Sigma)$, $O_2(^1\Delta)$, $O_3$, NO, $N_2O$, $NO_2$, $NO_3$ and $N_2O_5$, and UV photons.

Atomic nitrogen transports chemical energy from the generator to the contaminated sample, without significant recombination or losses on the walls of the decontamination enclosure. This energy is associated with a decontamination effect, where the nitrogen atoms and nitrogen-atom-induced species and photons come in contact with biological species of the contaminated sample 1. In some experiment, it has been shown that in some point of the contaminated region with concentration of atomic nitrogen around $10^{13}$ $cm^{-3}$, concentration of B. stéarothermophilus roughly decreased as a function of time t according to an exponential function of the type $e^{-t/to}$, where $t_o$ is of the scale of the minute. High decontamination activity is therefore obtained in the whole contaminated region, and not only close to the generators. Of course, decontamination with the inventive method is not limited to applications to B. Stéarothermophilus, but covers all kind of biological species to be destroyed.

Using $N_2$ as vector gas at atmospheric (or close to atmospheric) pressure and temperature enables a decontamination which has no harmful effect on the sample to be decontaminated, neither due to warmth nor to acid production. Exhaust gas arriving at the second end 2b of the decontamination enclosure can be filtered by an adapted filter 4, before leaving into the atmosphere, or can be fed back into the generator 3. Optionally, said exhaust gas is recycled by an adapted recycling unit 9 and fed back to intake duct 7, as shown on Fig. 1. The gas may freely exit into the atmosphere, because the vector and source gas being nitrogen, it causes no harm to the environment. The eventual amounts of $-N_yO_x$ or $-O_3$ in the exhaust can be controlled to less than a ppm. Suitable filters are already described in US 6 345 497, US 5 547 651, US 5 782 085 and US 5 640 845 for pollution control methods using atomic nitrogen, for the automotive industry.

It is possible to add many parallel generators connected to different entrance points of the first end of the contaminated region such as shown on Fig. 1, as well as a main entrance point through which a non-dissociated vector gas is fed into the decontamination region, without passing through a generator.

With reference to Fig. 2, a second embodiment of the method for decontamination is presented in which the method is used to decontaminate internal walls 10 of a contaminated region 2, in particular a room or ducts such as aeration conducts and the like. Fig. 2 accordingly represents an aeration conduct of diameter D of a few centimetres and a length L of some metres. Such ducts commonly have open sections 11 which are connected to different rooms 12 in a building. No decontamination enclosure is therefore needed, as decontamination enclosure is formed by said internal walls 10 of conducts.

Where it is possible to do so, the open sections are connected to a generator 3 of the kind used in the method described in Fig. 1. Open sections which are not connected to a generator could be left open, optionally through a filter, or are closed for the duration of the decontamination, so that a suitable gas flow rate could be maintained throughout duct 2. The vector gas is input through first end 2a of the aeration duct, and carries the decontamination gases output by the generators and containing the active species, throughout the duct. If necessary, decontamination gas and decontamination products are filtered in second end 2b of the duct, optionally recycled and fed back to first end 2a.

The working principles of the generator are now described with respect to the specific embodiment of Fig. 3a and 3b. Nevertheless, any generator able to produce nitrogen atoms in operative concentrations at those operative conditions is considered to be encompassed by the present document. In particular, generators described in aforementioned patents US 6 345 497, US 5 547 651, US 5 782085 and US 5 640 845 are suitable.

In the described method, the flow of nitrogen gas in the contaminated region has the following characteristics:

- a concentration of nitrogen atoms close to generator of $10^{15}$ $cm^{-3}$,
- a flow speed of 20 m/s, vector gas pressure close to atmospheric pressure, and
- a temperature of 300 K.

The present generator is adapted to provide with such a flow. The generator comprises two electrodes, $E_1$ and $E_2$ between which source gas flows from gas sources 6 at a given flow rate, as indicated by arrow 13 on Fig. 3. A voltage pulse is periodically applied from a voltage source 14 of power between 10 and 100 W, and a fast commuter 15 for providing short pulses between the two electrodes. These shorts pulse enable an efficient breakdown of the gas with a high voltage while limiting the transferred charge. The pulse comprises a voltage increase stage 17a, a steady state stage 17b, for example around 20 kV, a voltage decrease stage 17c, and an off-state stage 17d, as shown on Fig. 3b. Discharge frequency rate is for example around 30 kHz, in this particular embodiment. Discharge frequency and gas velocities are adapted to each other so that subsequent discharges are temporally independent with respect to each other.

If the voltage increase stage 17a occurs during a very short time, many energetic electrons will be produced in the gas which will be effective for molecular nitrogen dissociation and atomic nitrogen production. Nevertheless, if one increases the frequency rate at which such increase stages occur, rest species from the precedent discharge would still be present between the electrodes, the necessary voltage to be applied for the dissociation will decrease, which in turn will decrease the discharge efficiency.

A solution is to have an adapted gas flow between the electrodes, so that rest species are carried away with the non-activated species. Each pulse creates an ionisation path between the two electrodes which is subsequently moved with the gas.

A first discharge will thus follow a straight path 16a between the electrodes. With the gas flowing in the direction of arrow 13, the rest species of first discharge will be located around 16b when second discharge occurs. Path 16b will therefore be a preferential path for second discharge, as well as paths 16c and 16d for subsequent discharges. Each time, the spatial extent of the discharge is longer, and the voltage necessary-for gas breakdown will decrease, therefore having a negative effect on dissociation efficiency.

This phenomenon will continue until the ionised path from the precedent discharge is too far away and the discharge follows a new ionisation path straight between the two electrodes that maximises the voltage. If the gas flow rate is adapted, the path ionised by the first pulse is already "too far away" from the electrodes when the second pulse occurs, so that maximal voltage is obtained for all discharges.

Furthermore, screens can be placed between the electrodes in order to provide for spatial independence of the discharges, such as shown on Fig. 3c, where gas flows normal to the plane of the Figure as indicated by arrow 13 pointing towards us. Isolating screens 19, made from glass or ceramics, for example, separate many dissociation entities such as described previously with respect to Fig. 3a, so that discharges can take place independently.

Such discharge electrodes can readily be set in series or in parallel in the gas flow in order to provide for efficient nitrogen dissociation.

Such generators offer the following advantages:

- fast pick-up by vector gas, enabled by the small volume of atomic nitrogen generation region,
- generators easily set in series or parallel,
- ability to provide wide operative temperature and pressure ranges,
- ability to cool vector gas by expansion after the discharge,
- high reliability,
- performance comparable to beam electron techniques for lower costs,
- reduced electromagnetic noise.

In the method presented here, the atomic nitrogen high concentrations are obtained in the post-discharge of a nitrogen plasma, but any classical way of obtaining high nitrogen atom concentrations such as electron beam techniques, micro-waves or sparks, offering suitable atomic nitrogen concentrations gas flow rate and speeds at operative temperature and pressure could alternatively be used within the method.

## Claims

1. Method for decontamination of a contaminated region containing at least biological species to be destroyed, comprising:

   - an activation step during which a decontamination gas is obtained by dissociation in a generator of a source gas containing molecular nitrogen;
   - a decontamination step during which the decontamination gas containing at least atomic nitrogen is propagated in said contaminated region, said atomic nitrogen transporting chemical energy that contributes to the destruction of at least part of said biological species in said contaminated region;

   **characterized in that** :

   - said source gas contains at least 99% of molecular nitrogen in volume;
   - wherein said decontamination region is inside a decontamination enclosure comprising an intake end and an exhaust end, and wherein a vector gas is propagated into said decontamination enclosure from said intake end to said exhaust end, the decontamination gas forming at least part of said vector gas;
   - said source gas flows into a generator at a given flow rate, and wherein, during the activation step, said source gas is passed between two electrodes and is dissociated by discharge voltage applied at a given discharge frequency between said two electrodes, each discharge following a given discharge path between said two electrodes, said discharge frequency being adapted to said flow rate so that subsequent discharge paths are independent with respect to each other.

2. Method according to claim 1 wherein said source gas containing molecular nitrogen is at a pressure between 6666.1 Pa and 533.288 kPa (50 Torr and 4000 Torr).

3. Method according to claim 1 or 2 wherein a source gas generator produces said source gas containing at least 99% of molecular nitrogen in volume from atmospheric air.

4. Method according to any of claims 1 to 3, wherein said source gas contains over 99,95% $N_2$ in volume.

**5.** Method according to claims 1-4, wherein said decontamination gas is propagated into said decontamination enclosure in the vicinity of said intake end.

**6.** method according to any of claims 1-5, wherein said exhaust end is in communication with atmospheric air.

**7.** Method according to any of claims 1-6, wherein exhaust gas is collected in said exhaust end and is fed back to form at least part of vector gas

**8.** Method according to any of claims 1 to 7, wherein said vector gas comprises at least a gas taken among $O_2$ or air.

**9.** Method according to any of claims 1 to 10, wherein said decontamination gas is propagated in said contaminated region so that decontamination gas temperature is comprised between 220 and 400 K in said contaminated region.

**10.** Method according to any of claims 1-9 wherein, at the exhaust end, said decontamination gas contains decontamination products and wherein said decontamination products are filtered out of decontamination gas to provide for a cleaned decontamination gas.

**11.** Method according to any of claims 1 to 10, further comprising an input step wherein said source gas fed into said generator has at least one of the following characteristics:

- a flow speed comprised between 1 and 100 m/s,
- and a temperature comprised between 220K and 400K, and wherein, during activation step, said discharge frequency is comprised between 1 kHz and 200 kHz.

**12.** Method of decontamination of a contaminated region according to any of claims 1-11, comprising a placing step wherein said contaminated sample is placed within said decontamination enclosure, and wherein said contaminated region is a contaminated sample.

**13.** Method of decontamination of a contaminated region according to any of claims 5 to 12, said contaminated region being a duct, said duct comprising contaminated internal walls and at least a first and a second duct openings, wherein said first duct opening forms said intake end, said second duct opening forms said exhaust end, and wherein said internal wall form said decontamination enclosure, said decontamination gas being propagated from at least one duct opening.

**14.** Method of decontamination of a contaminated region according to claim 13, said duct containing further openings, said method comprising a setting step wherein said further openings are either sealed, connected to a generator, or provided with a filter.

**Patentansprüche**

**1.** Verfahren zur Dekontaminierung eines kontaminierten Bereichs, der wenigstens biologische Spezies enthält, die zu vernichten sind, umfassend:

- einen Aktivierungsschritt, während dem ein Dekontaminierungsgas durch Dissoziation in einem Generator eines Quellengases, das molekularen Stickstoff enthält, erhalten wird;
- einen Dekontaminierungsschritt, während dem das Dekontaminierungsgas, das wenigstens atomaren Stickstoff enthält, in den/dem kontaminierten Bereich verbreitet wird, wobei der atomare Stickstoff chemische Energie transportiert, die zur Vernichtung wenigstens eines Teils der biologischen Spezies in dem kontaminierten Bereich beiträgt;

**dadurch gekennzeichnet, dass**:

- das Quellengas wenigstens 99 Volumen-% molekularen Stickstoff enthält;
- wobei der Dekontaminierungsbereich innerhalb einer Dekontaminierungseingrenzung ist, die ein Einlassende und ein Auslassende umfasst, und wobei ein Vektorgas vom Einlassende zum Auslassende in die Dekontaminierungseingrenzung verbreitet wird, wobei das Dekontaminierungsgas wenigstens einen Teil des Vektorgases bildet;
- wobei das Quellengas mit einer bestimmten Strömungsgeschwindigkeit in einen Generator strömt, und wobei während des Aktivierungsschritts das Quellengas zwischen zwei Elektroden durchgeführt und durch Entladungsspannung, die bei einer bestimmten Entladungsfrequenz zwischen den beiden Elektroden angelegt wird, dissoziiert wird, wobei jede Entladung einem bestimmten Entladungsweg zwischen den beiden Elektroden folgt, und die Entladungsfrequenz derart an die Strömungsgeschwindigkeit angepasst wird, dass nachfolgende Entladungswege unabhängig in Bezug aufeinander sind.

**2.** Verfahren nach Anspruch 1, wobei das Quellengas, das molekularen Stickstoff enthält, bei einem Druck zwischen 6666,1 Pa und 533,288 kPa (50 Torr und 4000 Torr) ist.

**3.** Verfahren nach Anspruch 1 oder 2, wobei ein Quellengasgenerator das Quellengas erzeugt, das wenigstens 99 Volumen-% molekularen Stickstoff aus atmosphärischer Luft enthält.

**4.** Verfahren nach einem der Ansprüche 1 bis 3, wobei das Quellengas über 99,95 Volumen-% $N_2$ enthält.

**5.** Verfahren nach Anspruch 1 bis 4, wobei das Dekontaminierungsgas in der Nähe des Einlassendes in die Dekontaminierungseingrenzung verbreitet wird.

**6.** Verfahren nach einem der Ansprüche 1 bis 5, wobei das Auslassende in Verbindung mit atmosphärischer Luft ist.

**7.** Verfahren nach einem der Ansprüche 1 bis 6, wobei Abgas im Auslassende gesammelt und zurückgeführt wird, um wenigstens einen Teil von Vektorgas zu bilden.

**8.** Verfahren nach einem der Ansprüche 1 bis 7, wobei das Vektorgas wenigstens ein Gas umfasst, das unter $O_2$ oder Luft ausgewählt wird.

**9.** Verfahren nach einem der Ansprüche 1 bis 8, wobei das Dekontaminierungsgas derart in den/dem kontaminierten Bereich verbreitet wird, dass die Dekontaminierungsgastemperatur in dem kontaminierten Bereich zwischen 220 und 400 K liegt.

**10.** Verfahren nach einem der Ansprüche 1 bis 9, wobei am Auslassende das Dekontaminierungsgas Dekontaminierungsprodukte enthält, und wobei die Dekontaminierungsprodukte aus dem Dekontaminierungsgas gefiltert werden, um für ein gereinigtes Dekontaminierungsgas zu sorgen.

**11.** Verfahren nach einem der Ansprüche 1 bis 10, ferner umfassend einen Eingabeschritt, wobei das Quellengas, das in den Generator eingeführt wird, wenigstens eine der folgenden Charakteristiken aufweist:

- eine Strömungsgeschwindigkeit, die zwischen 1 und 100 m/s liegt, und
- eine Temperatur, die zwischen 220 K und 400 K liegt, und wobei während des Aktivierungsschritts die Entladungsfrequenz zwischen 1 Hz und 200 Hz liegt.

**12.** Verfahren zur Dekontaminierung eines kontaminierten Bereichs nach einem der Ansprüche 1 bis 11, umfassend einen Anordnungsschritt, wobei die kontaminierte Probe innerhalb der Dekontaminierungseingrenzung angeordnet wird, und wobei es sich bei dem kontaminierten Bereich um eine kontaminierte Probe handelt.

**13.** Verfahren zur Dekontaminierung eines kontaminierten Bereichs nach einem der Ansprüche 5 bis 12, wobei es sich bei dem kontaminierten Bereich um ein Rohr handelt, das Rohr kontaminierte Innenwände und wenigstens eine erste und eine zweite Rohröffnung umfasst, wobei die erste Rohröffnung das Einlassende bildet, die zweite Rohröffnung das Auslassende bildet, und wobei die Innenwände die Dekontaminierungseingrenzung bilden, und das Dekontaminierungsgas von wenigstens einer Rohröffnung verbreitet wird.

**14.** Verfahren zur Dekontaminierung eines kontaminierten Bereichs nach Anspruch 13, wobei das Rohr weitere Öffnungen enthält, und das Verfahren einen Einstellschritt umfasst, wobei die weiteren Öffnungen entweder abgedichtet, mit einem Generator verbunden oder mit einem Filter versehen werden.

## Revendications

**1.** Procédé pour décontaminer une région contaminée contenant au moins une espèce biologique devant être détruite, comprenant :

- une étape d'activation durant laquelle un gaz de décontamination est obtenu par dissociation dans un générateur d'un gaz source contenant de l'azote moléculaire ;
- une étape de décontamination durant laquelle le gaz de décontamination contenant au moins de l'azote atomique est propagé dans ladite région contaminée, ledit azote atomique transportant l'énergie chimique qui contribue à la destruction d'au moins une partie de ladite espèce biologique dans ladite région contaminée ;

**caractérisé en ce que** :

- ledit gaz source contient au moins 99 % en volume d'azote moléculaire ;
- dans lequel ladite région de décontamination se trouve à l'intérieur d'une enceinte de décontamination comprenant une extrémité d'admission et une extrémité d'échappement, et dans lequel un gaz vecteur est propagé dans ladite enceinte de décontamination depuis ladite extrémité d'admission vers ladite extrémité d'échappement, le gaz de décontamination formant au moins une partie dudit gaz vecteur ;
- ledit gaz source circule dans un générateur à un débit donné, et dans lequel, durant l'étape d'activation, ledit gaz source passe entre deux électrodes et est dissocié par une tension de décharge appliquée à une fréquence de décharge donnée entre lesdites deux électrodes, chaque décharge suivant un trajet de décharge donné entre lesdites deux électrodes, ladite fréquence de décharge étant adaptée audit débit de façon que des trajets de décharge subséquents soient indépendants les uns des autres.

2. Procédé selon la revendication 1, dans lequel ledit gaz source contenant de l'azote moléculaire est sous une pression comprise entre 6666,1 Pa et 533,288 kPa (entre 50 Torr et 4 000 Torr).

3. Procédé selon la revendication 1 ou 2, dans lequel un générateur de gaz source produit ledit gaz source contenant au moins 99 % en volume d'azote moléculaire à partir d'air atmosphérique.

4. Procédé selon l'une quelconque des revendications 1 à 3, dans lequel ledit gaz source contient plus de 99,95 % en volume de $N_2$.

5. Procédé selon les revendications 1 à 4, dans lequel ledit gaz de décontamination est propagé dans ladite enceinte de décontamination au voisinage de ladite extrémité d'admission.

6. Procédé selon l'une quelconque des revendications 1 à 5, dans lequel ladite extrémité d'échappement est en communication avec l'air atmosphérique.

7. Procédé selon l'une quelconque des revendications 1 à 6, dans lequel le gaz d'échappement est collecté dans ladite extrémité d'échappement et est réintroduit pour former au moins une partie du gaz vecteur.

8. Procédé selon l'une quelconque des revendications 1 à 7, dans lequel ledit gaz vecteur comprend au moins un gaz choisi parmi $O_2$ et l'air.

9. Procédé selon l'une quelconque des revendications 1 à 8, dans lequel ledit gaz de décontamination est propagé dans ladite région contaminée de façon que la température de gaz de décontamination soit comprise entre 220 et 400 K dans ladite région contaminée.

10. Procédé selon l'une quelconque des revendications 1 à 9, dans lequel, à l'extrémité d'échappement, ledit gaz de décontamination contient des produits de décontamination et dans lequel lesdits produits de décontamination sont retirés par filtration du gaz de décontamination pour former un gaz de décontamination nettoyé.

11. Procédé selon l'une quelconque des revendications 1 à 10, comprenant en outre une étape d'entrée dans laquelle ledit gaz source introduit dans ledit générateur a au moins l'une des caractéristiques suivantes :

    - un débit compris entre 1 et 100 m/s, et

    - une température comprise entre 220 K et 400 K

et dans lequel, durant l'étape d'activation, ladite fréquence de décharge est comprise entre 1 kHz et 200 kHz.

12. Procédé de décontamination d'une région contaminée selon l'une quelconque des revendications 1 à 11, comprenant une étape de placement dans laquelle ledit échantillon contaminé est placé à l'intérieur de ladite enceinte de décontamination, et dans lequel ladite région contaminée est un échantillon contaminé.

13. Procédé de décontamination d'une région contaminée selon l'une quelconque des revendications 5 à 12, ladite région contaminée étant un conduit, ledit conduit comprenant des parois internes contaminées et au moins une première et une deuxième ouvertures de conduit, dans lequel ladite première ouverture de conduit forme ladite extrémité d'admission, ladite deuxième ouverture de conduit forme ladite extrémité d'échappement, et dans lequel lesdites parois internes forment ladite enceinte de décontamination, ledit gaz de décontamination étant propagé depuis au moins une ouverture de conduit.

14. Procédé de décontamination d'une région contaminée selon la revendication 13, ledit conduit contenant d'autres ouvertures, ledit procédé comprenant une étape d'établissement dans laquelle lesdites autres ouvertures sont soit scellées, soit connectées à un générateur, soit dotées d'un filtre.

FIG.1.

FIG.2.

FIG.3a.

FIG.3b.

FIG.3c.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- FR 2790962 A
- US 3383163 A
- US 6030506 A
- WO 0054819 A
- WO 02070025 A
- US 6345497 B
- US 5547651 A
- US 5782085 A
- US 5640845 A

### Non-patent literature cited in the description

- *Sterilization of medical apparatus by cold plasmas,* 19 October 2001
- Sterilization of Medical Products in Low-Pressure Glow Discharges. *Plasma Physics Reports,* 2000
- STERILIZATION OF MEDICAL PRODUCTS IN LOW-PRESSURE GLOW DISCHARGES. PLASMA PHYSICS REPORTS. AMERICAN INSTITUTE OF PHYSICS